# EUROPÄISCHE PATENTSCHRIFT

(11) **EP 3 099 360 B1**
(45) Veröffentlichungstag und Bekanntmachung des Hinweises auf die Patenterteilung: **15.05.2019**
(21) Anmeldenummer: 15705188.9
(22) Anmeldetag: 20.01.2015
(51) Int. Cl.: A61M 11/00, A61M 15/00, A61M 15/08

(54) **PULVERINHALATOR UND PULVERINHALATIONSSET**
POWDER INHALER AND POWDER INHALATION SET
INHALATEUR DE POUDRE ET SET POUR L'INHALATION DE POUDRE

(30) Priorität: 30.01.2014 DE 102014001072; 17.04.2014 DE 102014005647
(43) Veröffentlichungstag der Anmeldung: 07.12.2016
(73) Patentinhaber: Beller, Klaus-Dieter, 79341 Kenzingen (DE)
(72) Erfinder: Beller, Klaus-Dieter, 79341 Kenzingen (DE)
(74) Vertreter: Mehl-Mikus, Claudia
(86) Internationale Anmeldenummer: PCT/EP2015/000093
(87) Internationale Veröffentlichungsnummer: WO 2015/113743

(56) Entgegenhaltungen:
- EP-B1- 2 015 812
- WO-A1-2011/154371
- WO-A2-2013/036881
- WO-A2-2014/006135
- DE-A1-102009 030 185
- DE-A1-102009 041 664
- GB-A- 2 270 293
- GB-A- 2 460 281
- US-A- 6 073 629

## Beschreibung

Die Erfindung betrifft einen Pulverinhalator und ein Pulverinhalationsset aus Pulverinhalator und Blisterelement.

Pulverinhalatoren zur sublingualen, nasalen und inhalativen Applikation pulverförmiger, feststofflicher Arzneimittel oder anderer Mitteln sind bekannt. Sie können als treibgasfreie Applikationsvorrichtungen ausgestaltet sein und setzen ein Aerosol durch den Inspirationsvorgang, respektive einen tiefen Inhalationsvorgang frei. Die Energie für die Dispergierung wird dabei durch den inspiratorischen Fluss gewonnen. Die pulverförmige Substanz ist dabei in einem Vorratsbehältnis enthalten; es sind auch spezielle Vorratsbehältnisse wie Blister bekannt. Je nach Art des Pulverinhalators wird der reine Wirkstoff oder es wird der Wirkstoff mit einem Träger, der ein unbedenklicher Hilfsstoff wie beispielsweise Lactose oder Glucose für adhährierte Wirkstoffpartikel ist, eingesetzt.

Bekannte Pulverapplikatoren bzw. Pulverinhalatoren können je nach Ausgestaltung bis zu drei verschiedene Sorten von Pulvern verabreichen. Einen solchen Pulverinhalator beschreibt die EP 1 769 818 B1: Dort wird ein treibgasfreier Pulverinhalator zur inhalativen Applikation pulverförmiger feststofflicher Arzneimittel eingesetzt, der es auch erlaubt, eine Pulverkombination zu inhalieren. Dies wird dadurch erreicht, dass der Pulverinhalator wenigstens zwei Vorratsbehältnisse aufweist, in denen unterschiedliche Pulver voneinander getrennt in mehreren Dosiereinheiten bevorratet sind. Die Pulver können entsprechend zusammengeführt und über ein gemeinsames Inhalationsrohr verabreicht werden.

Ebenfalls für die Inhalation zweier unterschiedlicher Pulver vorgesehen ist der Pulverinhalator aus DE 10 2005 046 645 B3, der für die unterschiedlichen Pulver ebenfalls wenigstens zwei Vorratsbehältnisse aufweist und es ermöglicht, dass diese getrennt voneinander in einer Dosiereinrichtung dosiert und anschließend während des Inhalierens miteinander vermischt werden.

Die Verabreichung auch unterschiedlicher Pulver kann als eine Einzeldosis erfolgen.

Um den Agglomerationsgrad und den Entleerungsgrad des Pulvers beim Inhalieren zu verbessern, weist der Pulverinhalator der DE 10 2005 046 644 B3 im Zuluftkanal eine wendel- oder spiralförmige Verwirbelungseinrichtung auf, um die Luft vor der Zufuhr zu dem Pulver zu verwirbeln und so eine feinere Verteilung des Pulvers im Luftstrom zu erreichen.

Ein Inhalator für Einzeldosen ist aus der DE 20 2011 103503 U1 bekannt. Der dort beschriebene Pulverinhalator für Kapseln umfasst ein Klingengehäuse und ein Mundstückgehäuse, sowie einen Kapselträger und mehrere Klingen. Der Pulverinhalator dort soll ohne komplizierte Vorbereitungen durch einen ungeübten Benutzer eingesetzt werden können. Der gesamte Inhalationsvorgang soll durch lediglich eine Schiebebewegung aktiviert werden können, dazu weist die Vorrichtung dort nur zwei zueinander bewegliche Gehäuseteile auf, durch deren Verschiebung zueinander zunächst die Einlegeöffnung für die Kapsel verschlossen und dadurch die Kapsel in einer Bohrung im Inhalator zurück gehalten wird. Im Inneren des Inhalators wird die Kapsel dann mit geeignet geformten, metallischen Klingen geöffnet.

Aus EP 2 015 812 B1 ist ein Pulverinhalator zur gleichzeitigen Verabreichung von mehreren Medikamenten bekannt, die in einem Einzeldosis-Blisterstreifen in verschiedenen Mulden vorliegen. Zum Einlegen des Blisters ist ein Mundstück des Pulverinhalators beweglich an einer Streifenauflage angesetzt. An der Streifenauflage des Pulverinhalators sind entsprechende Mulden zur Aufnahme der Blistermulden ausgeformt. Eine Deckfolie des eingelegten Blisters lässt sich bei geschlossenem Mundstück abziehen, indem die Deckfolie einen aus dem geschlossenen Pulverinhalator herausragenden umgeschlagenen Überlapp aufweist.

Während die meisten bekannten Inhalatoren aus einer Vielzahl von Bauteilen bestehen, was die Fertigung sehr teuer und aufwändig gestaltet, ist der oben genannte Inhalator DE 20 2011 103503 U1 bereits konstruktiv einfach und günstig fertigbar gestaltet; verlangt aber den Einsatz von Klingen und verfügt nur über einen kurzen, einfachen Transportweg um das Pulver aus der Kapsel mittels eingezogener Luft in ein entsprechendes Aerosol zu überführen.

WO 2011/154371 A1 offenbart einen Pulverinhalator mit sehr komplexem Aufbau, der aus einer Mehrzahl von Teilen besteht. Eine Vielzahl von Lufteinlassöffnungen ist in dem Deckel vorgesehen, der einen gebogenen Luftstromkanal schließt. Die Lufteinlassöffnungen erstrecken sich flächig über den Luftstromkanal, der einen Direktkanalabschnitt, der direkt zur Wirbelkammer führt, und einen Behälterkanalabschnitt aufweist, der die Aufnahmemulde passiert, in der ein Blister mit seiner Wirkstoffmulde aufgenommen ist. Eine vom Deckel in Richtung der Aufnahme weisende Rippe sorgt für eine Umlenkung des Luftstromkanals in die Wirkstoffmulde.

In WO 2013/036881 A2 ist ein dreiteiliger Pulverinhalator beschrieben, bei dem der Pulver-Luftauslassbereich zum Mundstück hin ein separates Bauteil ist. In einem zweiten Bauteil ist eine Torsionskammer ausgeformt, in die Lufteinlasskanäle, die in einem dritten Bauteil ausgeformt sind, nach Zusammenfügen der Bauteile münden. Das dritte Bauteil weist außerdem eine Auslassgitteröffnung auf, die auf der Torsionskammer zu liegen kommt und an der das separate Auslassstück befestigt wird. Dieser Inhalator ist zur Einmalverwendung vorgesehen, die Bauteile werden miteinander verklebt, ein Öffnen des Pulverinhalators nach der Verwendung ist nicht vorgesehen.

Der aus WO 2014/006135 A2 bekannte Pulverinhalator besteht aus gelenkig über ein Filmscharnier miteinander verbundenen Halbschalen, die in der zusammengefügten Anordnung einen Lufteinlassbereich mit einer 180°-Wende des Luftstroms vor der Pulveraufnahmemulde und ein Gitter im Auslassbereich zur Desagglomeration aufweisen.

Aus DE 10 2009 041 664 A1 ist ein Pulverinhalator für ein in einem Blister verpacktes Pulver bekannt. Der Pulverinhalator besteht aus zwei Gehäuseteilen, die in vorbestimmter gegenseitiger Lage in einen aktivierten Zustand zusammenfügbar sind. Das Gehäuse bildet dabei einen an einem Einströmende und an einem Ausströmende offenen Luftströmungskanal aus und weist eine Einrichtung zur Aufnahme der Blistermulde mit dem pharmazeutischen Pulver sowie eine Vorrichtung zum Öffnen des Blisters auf, die beim Zusammenfügen der Gehäuseteile aktiviert wird. Das eine Gehäuseteil, in dem die Einrichtung zur Aufnahme der Blistermulde ausgebildet ist, ist ein längliches, flaches Unterteil. Das zweite Gehäuseteil mit der Vorrichtung zum Öffnen der Blistermulde ist ein längliches, flaches Oberteil, wobei die Vorrichtung zum Öffnen der Blistermulde als ein Vorsprung ausgebildet ist, mit dem die Deckfolie des Blisters beim Zusammenfügen der Gehäusteile durchstoßen wird. Wenigstens ein Gehäuseteil weist an seinen Längsrändern vorstehende Leisten auf, die im zusammengefügten Zustand der Gehäuseteile im Zusammenwirken mit dem anderen Gehäuseteil den Luftströmungskanal an seinen Längsseiten begrenzen.

GB 2 270 293 A beschreibt einen Pulverinhalator aus zwei Halbschalen, von denen die obere Halbschale mit Membranen verschlossene Bohrungen aufweist, in denen jeweils eine Pulverdosis vorliegt. Die Membran wird durch Eindrücken einer Kappe mit Schneidkante geöffnet, sodass das Pulver in den Pulverfreigabebereich gelangt. Der Inhalator weist gewundene Zuluftkanäle auf, die sich von den Lufteinlassöffnungen zu dem Pulverfreigabebereich erstrecken und für Luftverwirbelung sorgen.

Der in GB 2460281 beschriebene Inhalator besteht aus mehreren aufeinander laminierten Schichten, sodass er die Größe einer Kreditkarte aufweist. Die mittlere Schicht weist Ausschnitte für den Lufteinlassbereich, eine verjüngende Luftpassage, die Medikamentenkammern und den Luftauslassbereich auf. In der Deckschicht sind ein Lufteinlass, der über dem Lufteinlassbereich der mittleren Schicht zu liegen kommt, und Entlüftungsöffnungen, die über jeder Medikamentenkammer zu liegen kommen. Zwischen der mittleren und der Bodenschicht kann eine weitere Schicht mit einer Ausnehmung unter der Luftpassage in der mittleren Schicht vorgesehen sein. Diese Ausnehmung, in der das Medikament vorliegt, wird durch einen umgeklappten Folienstreifen versiegelt, der durch den Lufteinlass herausragt und zum Öffnen der Ausnehmung abgezogen werden kann.

Ausgehend von diesem Stand der Technik ist es Aufgabe der vorliegenden Erfindung, einen noch einfacher fertigbaren Inhalator zu schaffen, der auch bei mehrfacher Verwendung hygienisch einwandfrei bleibt.

Diese Aufgabe wird mit dem Pulverinhalator mit den Merkmalen des Anspruchs 1 gelöst.

Entsprechend ergibt sich weiter die Aufgabe, ein Pulverinhalationsset zu schaffen, das den Inhalator samt Wirkstoff direkt einsetzbar bereitstellt.

Diese Aufgabe wird durch das Pulverinhalationsset mit den Merkmalen des Anspruchs 13 gelöst.

Weiterbildungen der Vorrichtung und des Verfahrens sind in den Unteransprüchen ausgeführt.

Ein erfindungsgemäßer Pulverinhalator umfasst zwei Halbschalen, die gelenkig miteinander verbunden sind oder verbunden werden können. Die beiden Halbschalen umschließen in einer zusammengefügten Anordnung einen Lufteinlassbereich, einen Pulverdepositions- und -freigabebereich und einen Auslassbereich, durch die ein Fluidpfad verläuft. In dem Lufteinlassbereich weist zumindest eine der Halbschalen eine oder mehrere Lufteinlassöffnung(en) auf. Ferner liegt in dem Lufteinlassbereich zumindest eine Luftverwirbelungsstruktur vor, die den Fluidpfad zwischen der/den Lufteinlassöffnung(en) und dem Pulverdepositions- und -freigabebereich definiert, in dem eine der beiden Halbschalen wenigstens eine Pulveraufnahmemulde aufweist. Durch die Luftverwirbelungsstruktur wird die Luft in dem Lufteinlassbereich verwirbelt, so dass durch den in den Pulverdepositions- und -freigabebereich zugeführten verwirbelten Luftstrom das Pulver vollständig aus der Pulveraufnahmemulde ausgetragen und bereits zumindest teilweise desagglomeriert wird. Beide Halbschalen sind im Auslassbereich, der zumindest eine Desagglomerationsstruktur aufweist, zu einem Auslass ausgebildet. Auf diese Weise bilden die beiden Halbschalen im zusammengefügten Zustand quasi ein Strömungsgehäuse für den Fluidpfad zwischen den Lufteinlassöffnungen und dem Auslass, so dass das Aerosol, das beim Einatmen in dem Pulverdepositions- und -freigabebereich aus zu inhalierendem Pulver, das in der Pulveraufnahmemulde bereitgestellt wird, und Atemluft gebildet wird, durch den Auslass vom Anwender inhaliert werden kann. Der Auslass des Pulverinhalators kann als Mundstück, etwa als ein Schnabel geformt sein, oder zur Inhalation durch die Nase als ein Nasenstück, etwa als ein gebogenes Nasenrohr.

Es werden vorteilhaft lediglich zwei Bauteile benötigt, um durch die gelenkige Anordnung beide Halbschalen zu dem Pulverinhalator zusammenzufügen, ohne dass es weiterer Bauteile bedarf. Die Halbschalen des Pulverinhalators können so vorteilhaft als Spritzgussbauteile gefertigt werden, was die Herstellung besonders kostengünstig macht. Durch die gelenkige Verbindung der Halbschalen kann der Pulverinhalator einfach geöffnet und geschlossen werden, was das Sauberhalten des Pulverinhalators bei Mehrfachnutzung vereinfacht. Zudem unterstützen die Scharniere das richtige Zusammenfügen der Halbschalen durch den Anwender.

Um den Pulverinhalator geeignet handhaben zu können und ihn überall hin mitnehmen zu können, ist dieser erfindungsgemäß aus zwei flachen Halbschalen mit einer länglichen, im Wesentlichen rechteckigen, trapezförmigen, tropfenförmigen oder ovalen Basis ausgebildet. Die Schale ergibt sich, da die rechteckige oder andersförmige Basis durch eine Wand bzw. Wandabschnitte berandet ist. Die länglich ausgebildeten Halbschalen weisen daher eine Längsachse auf, an deren beiden Enden zum einen die Lufteinlassöffnungen und zum anderen der Auslass vorgesehen sind. So sind die Lufteinlassöffnungen an einem von der Auslassseite abgewandten Wandabschnitt einer oder beider Halbschalen angeordnet; vorteilhaft ist es die Halbschale, die nicht die Pulveraufnahmemulde trägt. Dies ist dadurch begründet, dass mit der Gesamtgestaltung der für einen optimalen Strömungsverlauf geformte Fluidpfad ausgebildet werden soll.

"Fluidpfad" meint hierin den Weg, den zunächst die Luft allein und nach Mittragen des Pulvers durch die Luft das Aerosol zurücklegt. Das Aerosol entsteht, wenn der in der Mulde vorliegende, portionierte pulverförmige Wirkstoff durch inhalierte Luft mitgenommen und auf der nachfolgenden Diffusorstrecke hinreichend vermischt und zerstäubt wird. Um einen idealen, vorzugsweise Venturirohr-ähnlichen Kanal zu bilden, in dem der Fluidpfad lokalisiert ist, weist erfindungsgemäß wenigstens eine der Halbschalen, bevorzugt aber beide, zumindest zwei Leitstege auf, die sich von dem Wandabschnitt, an dem die Lufteinlassöffnungen vorliegen, die sich an den Wandabschnitt mit den Lufteinlassöffnungen anschließen, bis hin zu der Pulveraufnahme erstrecken. Dabei reichen die Leitstege einer Halbschale bezüglich ihrer Höhe bis zu der anderen Halbschale oder - wenn beide Halbschalen Leitstege aufweisen - bis zu den Leitstegen der anderen Halbschale, sodass jeweils zwei nebeneinander benachbarte Leitstege einen Zuluftkanal ausbilden, der zu der Pulveraufnahmemulde hin einen sich verjüngenden Querschnitt aufweist, und den Fluidpfad in dem Lufteinlassbereich begrenzen.

Die Luft wird durch die Querschnittsverjüngung trichterförmig komprimiert und durch die in dem Luftkanal angeordnete Luftverwirbelungsstruktur, die Strömungshindernisse für den entlang dem Fluidpfad strömenden Luftstrom bildet, in Torsion versetzt, so dass der durch Einatmen erzeugte Luftstrom eine höchste Geschwindigkeit und Turbulenz an der "Trichterspitze" aufweist, wenn er die Pulveraufnahmemulde erreicht. Diese ist erfindungsgemäß beidseitig durch Führungswände eingefasst, die sich von den äußersten Längsstegen erstrecken den Luftstrom aus dem Lufteinlassbereich durch die Pulveraufnahmemulde leiten. Dadurch wird das Pulver aus der Pulveraufnahmemulde vollständig mitgenommen. Ein optimaler Strömungsverlauf wird damit unabhängig von der Art und Tiefe der Einatmung des Anwenders sichergestellt.

Um einen in einer Mulde eines Blisterelements enthaltenen Wirkstoff freizusetzen, wenn das Blisterelement mit der Mulde in der Pulveraufnahmemulde des Pulverinhalators aufgenommen ist, weist in dem Pulverdepositions- und -freigabebereich die Halbschale, die nicht die Pulveraufnahmemulde(n) aufweist, einen Stempel an einer entsprechenden gegenüberliegenden Stelle auf. Dieser Stempel ist mittels eines elastischen Einsatzes in dieser Halbschale in Richtung der zumindest einen Pulveraufnahmemulde bewegbar eingesetzt und als Dorn zur Öffnung einer Wirkstoffmulde des eingelegten Blisterelements ausgebildet. So kann der Stempel durch Druck von außen an der Stelle des elastischen Einsatzes in Richtung der Pulveraufnahmemulde(n) zum Durchstechen der Blisterfolie bewegt werden, und kehrt bei Nachlassen des äußeren Drucks infolge der Rückstellkraft des elastischen Einsatzes wieder in seine ursprüngliche Position zurück.

Alternativ oder zusätzlich zu dem Dorn ist an einer der Halbschalen an einem Seitenwandabschnitt auf Höhe der Pulveraufnahmemulde eine Aussparung vorgesehen, deren Breite und Höhe der Breite und Höhe eines Blisterelements entsprechen, das zum Einlegen in diesen Pulverinhalator vorbestimmt ist. Der Pulverinhalator an sich kann entsprechend ausgestaltet sein, dass er der Aufnahme bestimmter Blisterelemente dient. Erfindungsgemäß weist der Pulverinhalator mindestens eins der oben beschriebenen Merkmale des Dorns und der Aussparung.

Geeigneter Weise kann die Aussparung eine Höhe haben, die höher ist als die Höhe des Blisterelements. Höhe bezieht sich hierbei auf die Höhe der Trägerfolie und nicht auf die Höhe der Medikamentenmulde. Die zweite Halbschale, die nicht die Aussparung hat, weist an den Seitenwandabschnitt, der bei einer zusammengefügten Anordnung der Halbschalen über der Aussparung zu liegen kommt, einen Überstand auf. Dieser ist dazu ausgebildet, die Aussparung zu verschließen, wenn ein entsprechendes Blisterelement in den Pulverinhalator aufgenommen ist, d. h. dass die Blisterfolie durch die Aussparung aus dem Inhalator ragt, und der Überstand an der Blisterfolie anliegt. Die Aussparung kann so vorgesehen sein, dass der Inhalator für Rechts- oder Linkshänder ausgelegt ist.

Durch das Verschließen der Aussparung nach Einlegen des Blisterelements wird verhindert, dass an unerwünschter Stelle Luft beim Inhalieren in den Inhalator eingezogen wird.

Ist eine Ausführungsform des Pulverinhalators vorgesehen, mittels dessen gleichzeitig zwei oder mehr pulverförmige Medikamente inhaliert werden sollen und dementsprechend zwei oder mehr Pulveraufnahmemulden in einer der Halbschalen vorliegen, so sind die Leitstege derart angeordnet, dass jeweils zumindest ein Zuluftkanal zu jeder der Pulveraufnahmemulden führt.

Die in dem Zuluftkanal zwischen zwei benachbarten Leitstegen angeordnete Luftverwirbelungsstruktur - d. h. im Falle mehrerer Zuluftkanäle liegen auch mehrere Luftverwirbelungsstrukturen vor - kann als ein elliptischer Kegelstumpf geformt sein, entlang dessen Mantelfläche eine oder mehrere wendelartige Erhebung verlaufen. Im einfachsten Fall kann diese schneckenartige Luftverwirbelungsstruktur einfach eingelegt sein, vorzugsweise jedoch kann der Kegelstumpf über seine Basisfläche an dem von der Auslassseite abgewandten Wandabschnitt einer der Halbschalen angelenkt sein, so dass er etwa zu Reinigungszwecken bei geöffneten Halbschalen ausgeschwenkt werden kann. Besonders bevorzugt kann diese gelenkige Verbindung durch zumindest ein Filmscharnier unverlierbar realisiert sein, so dass die Luftverwirbelungsstruktur einstückig mit einer der Halbschalen hergestellt werden kann.

Alternativ zu dieser Luftverwirbelungsschnecke kann eine erfindungsgemäße Luftverwirbelungsstruktur durch mehrere Traversstege gebildet werden, die zwischen benachbarten Leitstegen verlaufen, wobei die Traversstege in beiden Halbschalen derart angeordnet sind, dass sie in der zusammengefügten Anordnung gegensinnig verlaufen, d. h. nicht übereinander zu liegen kommen, sondern einander kreuzen. Durch diese Strömungshindernisse verläuft der Fluidpfad von den Lufteinlässen abwechselnd in beiden Halbschalen bis zur Pulveraufnahmemulde und wird so in Torsion und Turbulenz versetzt.

Die gelenkige Verbindung der beiden Halbschalen kann beispielsweise durch ein Scharnier an benachbarten Seitenwänden oder durch ein Schwenkgelenk bereitgestellt werden. Durch das Scharnier lassen sich die Halbschalen in einer Klappbewegung zum Öffnen und Schließen um eine an der Kante vorliegende Längsachse verschwenken, während das Schwenkgelenk, das bevorzugt im Bereich zwischen einer Seitenwand und dem von der Auslassseite abgewandten Wandabschnitt angeordnet sein kann, ein Verdrehen der beiden Halbschalen gegeneinander ermöglicht. So gestattet die Ausführungsform mit Scharnier, dass Elemente im Inneren des Pulverinhalators wie z. B. die Desagglomerationsstrukturen über die Teilungsebene der jeweiligen Halbschale hinaus ragen können, solange sie in der zweiten Halbschale Aufnahme finden. Wird ein Schwenkgelenk eingesetzt, so ist darauf zu achten, dass die jeweiligen Strukturen nicht über die Wandung der Halbschale hinaus ragen.

Die Desagglomerationsstruktur bzw. jede Desagglomerationsstruktur im Auslassbereich kann durch eine Gruppe aus ringförmig um einen Zylinder angeordneten Profilelementen gebildet werden, wobei sich sowohl Zylinder als auch die Profilelemente jeder Desagglomerationsstruktur zwischen den beiden Halbschalen erstrecken. Je nach Ausführungsform der gelenkigen Verbindung der beiden Halbschalen kann/können die Desagglomerationsstruktur(en) an einer der beiden Halbschalen angeordnet sein oder, wenn mehr als eine Desagglomerationsstruktur vorliegt, an unterschiedlichen Stellen im Auslassbereich auf beide Halbschalen verteilt sein. Für die Ausführungsform mit Schwenkgelenk wird/werden die Desagglomerationsstruktur(en) durch zwei Halbstrukturen gebildet, die nicht über die Teilungsebene der Halbschalen ragen und von denen jeweils eine Halbstruktur an jeder Halbschale angeordnet ist, so dass die Halbstrukturen in der zusammengefügten Anordnung der Halbschalen die Desagglomerationsstruktur bilden. Die Desagglomerationsstrukturen dienen als Strömungshindernisse dazu, dass beim Transport des Pulvers und der Mitnahme durch den Luftstrom ggf. größere Pulverpartikel durch die Strömungshindernisse zerschlagen und damit verkleinert werden. Ferner kann dadurch gegebenenfalls der Wirkstoff von einer Trägersubstanz getrennt werden. Es können weitere, beliebig geformte mechanische Widerstände, respektive Strömungswiderstände, als Desagglomeratoren positioniert werden, um den Fluidpfad bedarfsgerecht und der Spezifikation des Medikaments angepasst auszubilden. In Frage kommen hier bspw. auch Stege, flügel- oder wallförmige sowie pollerförmige Strukturen.

Die Gesamtgestaltung des Fluidpfads dient der optimalen Strömung und Beschleunigung des inspirierten Luftstroms und der verbesserten Mitnahme und Zerstäubung des pulverförmigen Wirkstoffs. Es soll insbesondere erreicht werden, dass die Pulveraufnahmemulde von dem darin befindlichen Pulver mit einem Atemzug komplett entleert wird.

Die freie Gestaltung des Profils des Fluidpfads und der entsprechenden Strömungshindernisse bzw. Desagglomeratoren ermöglicht die Anpassung des Inhalators für verschiedene inspirationsgeeignete Wirkstoffe, die insbesondere Arzneimittel sein können. So können trockene, pulverförmige Arzneimittel im Strom in Partikel definierter Größe dispergiert werden.

Der erfindungsgemäße Inhalator ist durch seine Gestaltung und durch die Möglichkeit, ihn aufzuklappen und zu reinigen, besonders hygienisch handhabbar. Er ist ohne Klingen, Federn oder Hebel ausgebildet. Bei der Herstellung und Applikation von Pulveraerosol spielen die Adhäsion und die Reibung im Inhalator eine bedeutende Rolle; Adhäsion und Reibung zwischen Wirkstoff und Inhalator können durch die Fluidpfadführung überwunden werden, um zu verhindern, dass die Pulverteilchen an der Inhalatorinnenfläche adhäsiv anlagern. Die Strömungshindernisse im Luftzufuhrbereich und Auslassbereich sind so gestaltet, Geschwindigkeit und Turbulenz im Luftstrom zu optimieren, um eine bestmögliche Pulverfreisetzung und Desagglomeration zu erreichen, was zu einer verbesserten Inhalationsleistung führt.

Weiter kann die Halbschale, die die Pulveraufnahmemulde aufweist, wenigstens ein Anschlagselement aufweisen, das der Berandung eines zur Aufnahme in den Pulverinhalator vorbestimmten Blisterelementes dient. Damit kann das Blisterelement, wenn es eingelegt ist, nicht verrutschen.

Eine Ausführungsform sieht vor, dass das Öffnen der Wirkstoffmulde des Blisterelements nicht durch Perforation mittels eines als beweglicher Dorn ausgeführten Umlenküberstands, sondern durch Abziehen einer Deckelfolie erfolgt. Diese ragt mittels eines umgeschlagenen Überlapps durch die Aussparung aus dem zusammengefügten Pulverinhalator. Um das eingelegte Blisterelement beim Abziehen der Deckelfolie in dem Pulverinhalator festzuhalten, können beide Halbschalen an den Seitenwandabschnitten auf Höhe der Pulveraufnahmemulde gegenüber der Aussparung und gegenüber dem Überstand eine Riffelung aufweisen, deren Breite der Breite eines zum Einlegen in den Pulverinhalator vorbestimmten Blisterelements entspricht, so dass dieses zwischen den geriffelten Wandabschnitten in der zusammengefügten Anordnung des Pulverinhalators gehalten wird.

Alternativ kann vorgesehen sein, an der Halbschale, die die Pulveraufnahme aufweist, einen Überstand, etwa einen kuppelförmigen Überstand, bereitzustellen, der dazu ausgebildet ist, mit einer korrespondierenden Ausnehmung des Blisterelements in Eingriff gebracht zu werden, das dazu vorgesehen ist, in einen erfindungsgemäßen Pulverinhalator eingelegt zu werden.

Der Überstand oder die Riffelung dient als Widerlager beim Abziehen der Deckelfolie an dem aus dem geschlossenen Pulverinhalator ragenden Überlapp, so dass die Wirkstoffmulde des Blisterelements sicher in der Pulveraufnahmemulde verbleibt.

Im Bereich der Pulveraufnahmemulde kann wenigstens eine der beiden Halbschalen aus transparentem Kunststoff gefertigt sein, um das Hineinsehen in die Pulveraufnahmemulde zu ermöglichen und es damit zu erlauben, visuell zu überprüfen, ob das Pulver vollständig aus der Pulveraufnahmemulde entleert wurde, nachdem ein Inhalationsvorgang abgeschlossen ist.

An den Halbschalen sind geeigneter Weise Rastmittel zum Zusammenfügen vorgesehen; die Ausbildung derartiger Rastmittel ist dem Fachmann bekannt. Die Rastmittel können auf den entsprechenden Rändern der Halbschalen vorgesehen sein. Geeigneter Weise verfügen beide Halbschalen, gegebenenfalls auch angelenkte schneckenartige einlegbare Luftverwirbelungsstruktur, über Rastmittel, so dass diese Elemente durch Zusammenklappen einfach miteinander verrastet und gekoppelt werden können.

Weiterer Gegenstand der Erfindung ist ein Pulverinhalationsset, das aus dem erfindungsgemäßen Pulverinhalator einerseits und zum anderen aus einem entsprechenden Blisterelement besteht, das zur Aufnahme in den Pulverinhalator dimensioniert ist. Ein solches Blisterelement hat eine Trägerplatte oder -folie mit einer Mulde, in der das Pulver aufgenommen ist, respektive der inhalierbare Wirkstoff. Ein passendes Blisterelement kann aber auch zwei oder mehr Mulden mit unterschiedlichen, gleichzeitig zu inhalierenden Wirkstoffpulvern aufweisen. Das Blisterelement verfügt ferner über eine Deckelplatte oder -folie, die die Mulde(n) solange verschließt, wie das Pulver in den Blister bevorratet werden soll. Die Mulde(n) des Blisterelements ist/sind in Größe und Position entsprechend zur Aufnahme in die Pulveraufnahmemulde(n) des Pulverinhalators ausgebildet.

Um durch Öffnen der Mulde des Blisterelements das Pulver freizusetzen, wenn der Inhalationsvorgang durchgeführt werden soll, kann die Deckelplatte von der Trägerplatte und damit von der Wirkstoffmulde abgelöst werden, indem die Deckelplatte beispielsweise mit einem umklappbaren Überlapp ausgebildet ist, der bei in den Pulverinhalator eingelegtem Blisterelement durch die Aussparung in einem Seitenwandabschnitt auf Höhe der Pulveraufnahmemulde aus dem Pulverinhalator herausragt und abgezogen werden kann.

Eine alternative Ausführungsform sieht ein einfacheres Blisterelement vor, das nicht mit einem Abschnitt der Deckelplatte aus dem Pulverinhalator ragen muss, sondern lediglich passgenau mit der Wirkstoffmulde in die Pulveraufnahmemulde des Pulverinhalators eingelegt wird. Gegebenenfalls kann in dieser Ausführungsform auch ein kapselartiges Blisterelement oder eine speziell ausgebildete Kapsel eingesetzt werden. Das Öffnen der Wirkstoffmulde erfolgt hierbei nicht durch Abziehen der Deckelplatte sondern durch Perforation derselben. Ein dafür konzipierter erfindungsgemäßer Pulverinhalator ist mit einem elastisch bewegbaren und als Dorn ausgebildeten Stempel gegenüber der Pulveraufnahmemulde ausgebildet.

Es kann auch vorgesehen sein, dass ein erfindungsgemäßer Pulverinhalator mit beiden Öffnungsmechanismen ausgestattet ist, so dass beide Blisterarten geöffnet werden können.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- Fig. 1: eine perspektivische Ansicht eines erfindungsgemäßen Pulverinhalators aus zwei über Scharnier verbundenen Halbschalen in geöffnetem Zustand mit angelenkter Luftverwirbelungsstruktur,
- Fig. 2: eine Ansicht entsprechend Fig. 1 mit eingelegtem Blister,
- Fig. 3: eine perspektivische Ansicht des Pulverinhalators aus Fig. 1 mit eingelegter Luftverwirbelungsstruktur,
- Fig. 4: eine Längsschnittansicht durch den geschlossenen Pulverinhalator ohne Blister,
- Fig. 5: eine perspektivische Ansicht der zwei Halbschalen eines weiteren erfindungsgemäßen Pulverinhalators mit Schwenkgelenkverbindung,
- Fig. 6: eine perspektivische Ansicht des Pulverinhalator mit den zwei Halbschalen aus Fig. 5 in halbgeöffneter Position,
- Fig. 7: eine Längsschnittansicht den Pulverinhalator aus Fig. 6 in geschlossenem Zustand ohne Blister,
- Fig. 8: schematische Draufsicht auf die Luftverwirbelungsstruktur aus Traversstegen des Pulverinhalators aus Fig. 6/7,
- Fig. 9: eine perspektivische Draufsicht auf einen erfindungsgemäßen Pulverinhalator in geschlossenem Zustand mit Mundstück als Auslass,
- Fig. 10: eine perspektivische Draufsicht auf einen erfindungsgemäßen Pulverinhalator in geschlossenem Zustand mit Nasenstück als Auslass.

Die erfindungsgemäße Vorrichtung bezieht sich auf einen Pulverinhalator zur inhalativen Applikation durch Mund oder Nase eines in einem Blister bevorrateten pulverförmigen Wirkstoffs, der ein Medikament sein kann, aber auch ein Wirkstoff, der nicht zwingend als Medikament definiert ist, und der inhalativ von einer Person aufgenommen wird.

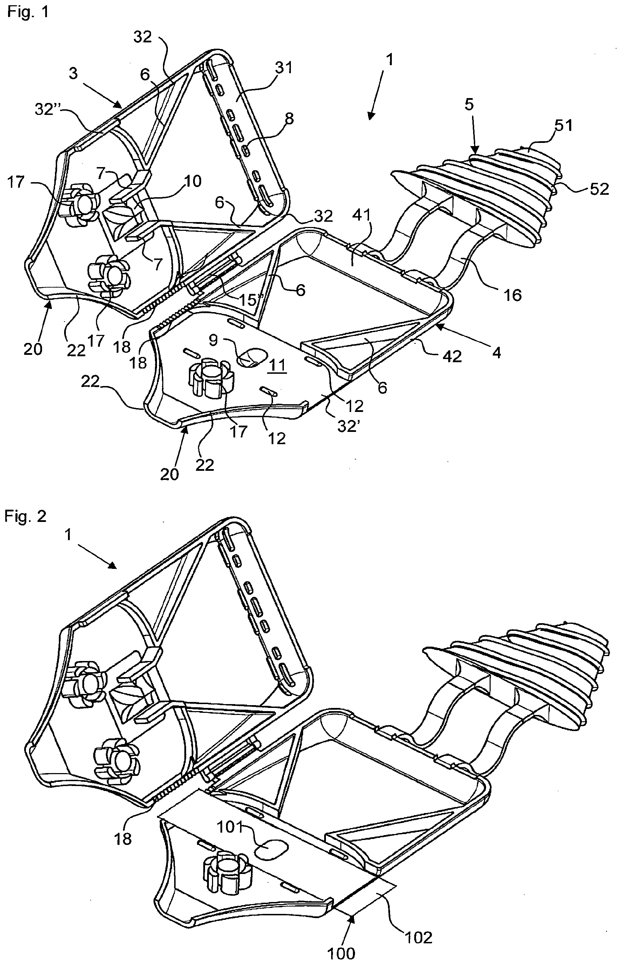

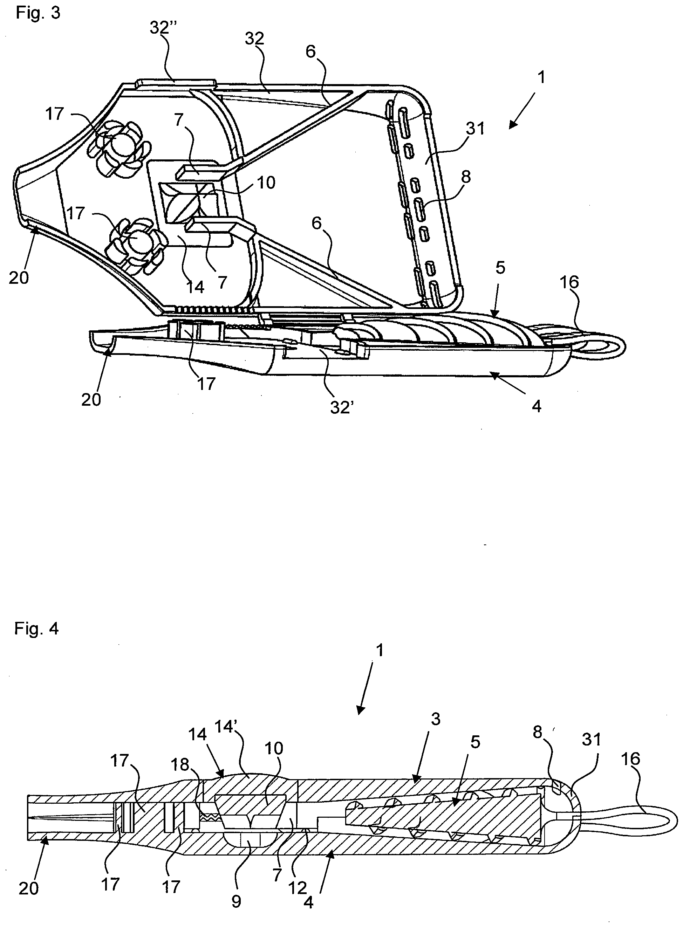

Fig. 1 zeigt zwei Halbschalen 3,4 eines erfindungsgemäßen Pulverinhalators 1 zur inhalativen Applikation, wobei die Halbschalen 3 und 4 über ein Scharnier 15" gelenkig verbunden sind. Eine der Halbschalen, vorliegend die untere Halbschale 4 ist über ein Filmscharnier 16 mit einer schneckenartigen Luftverwirbelungsstruktur 5 verbunden, die aus einem elliptischen kegelstumpfförmigen Grundkörper 51 besteht, um den sich eine Erhebung 52 windet, so dass der Luftstrom von den Einlassöffnungen 8 der Wendel 52 folgen muss und dadurch in Turbulenz und Rotation versetzt wird. Nach Einklappen der Luftverwirbelungsstruktur 5 in den Raum zwischen den Leitstegen 6 (vgl. **Fig. 3**) bilden die Halbschalen 3,4 in zusammengeklapptem Zustand den betriebsbereiten Pulverinhalator. Um den Pulverinhalator 1 in betriebsbereiten Zustand zu versetzen, wird an einer dafür vorgesehenen Stelle das Blisterelement 100 eingelegt (vgl. **Fig. 2**). Nach Einklappen der Luftverwirbelungsstruktur 5 und Zusammenklappen der beiden Halbschalen 3 und 4 (vgl. **Fig. 4**) ist der Pulverinhalator 1 einsatzbereit, es muss lediglich noch die Medikamentenmulde 101 des Blisterelements 100 geöffnet werden.

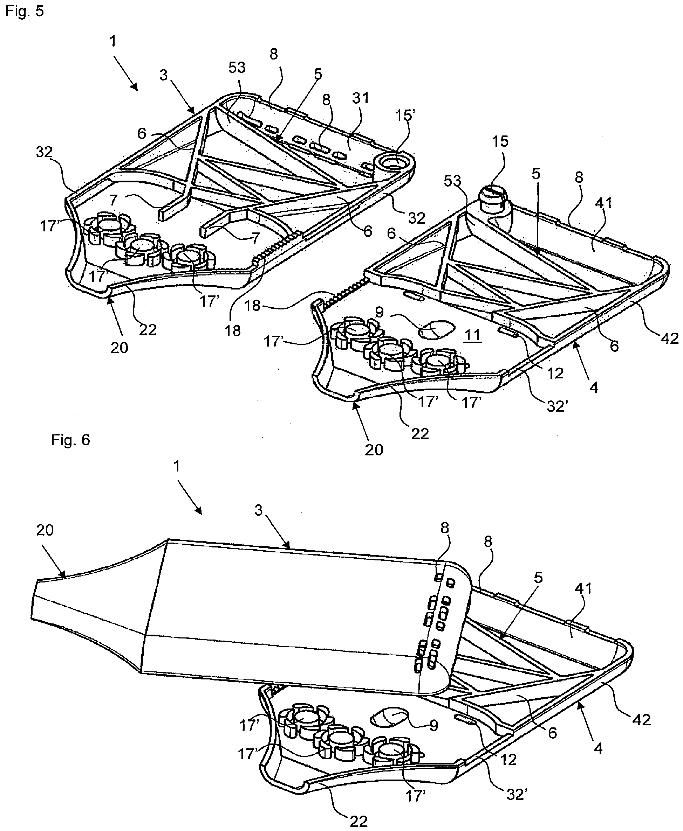

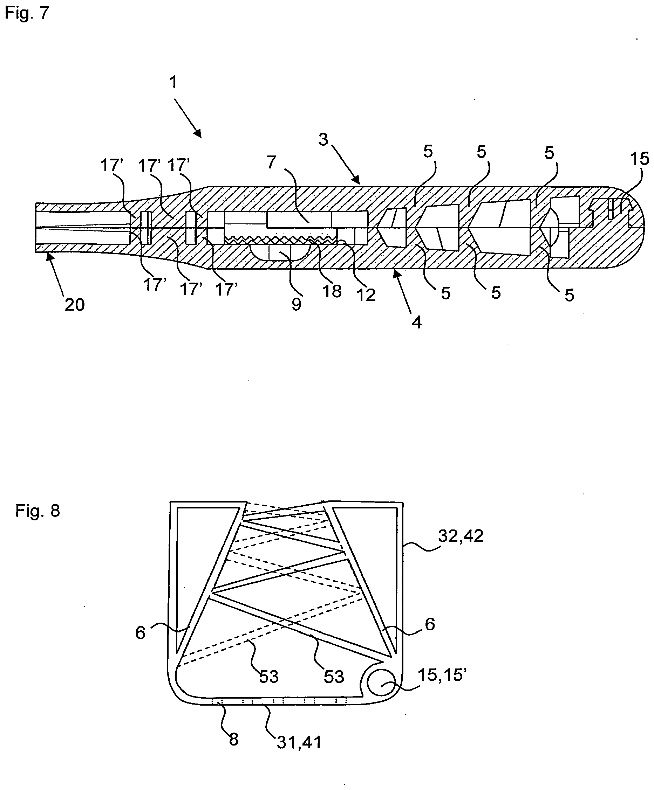

**Figuren 5 bis 8** zeigen einen weiteren erfindungsgemäßen Pulverinhalator 1 mit einer alternativen Luftverwirbelungsstruktur 5 und einem Drehgelenk 15,15' als gelenkige Verbindung der beiden Halbschalen 3 und 4. Hierbei wird die Luftverwirbelungsstruktur 5 durch Traversstege 53 gebildet, die in einer Zickzacklinie zwischen den Leitstegen 6 jeder Halbschale 3,4 verlaufen, so dass die Traversstege 53 in der zusammengefügten Anordnung der Halbschalen 3,4 einander kreuzen (vgl. **Fig. 8**) und so den Luftstrom von den Lufteinlassöffnungen 8 abwechselnd in die untere Halbschale 4 und die obere Halbschale 3 mit jeweiligem Richtungswechsel führen, so dass auch hier Turbulenzen erzeugt werden.

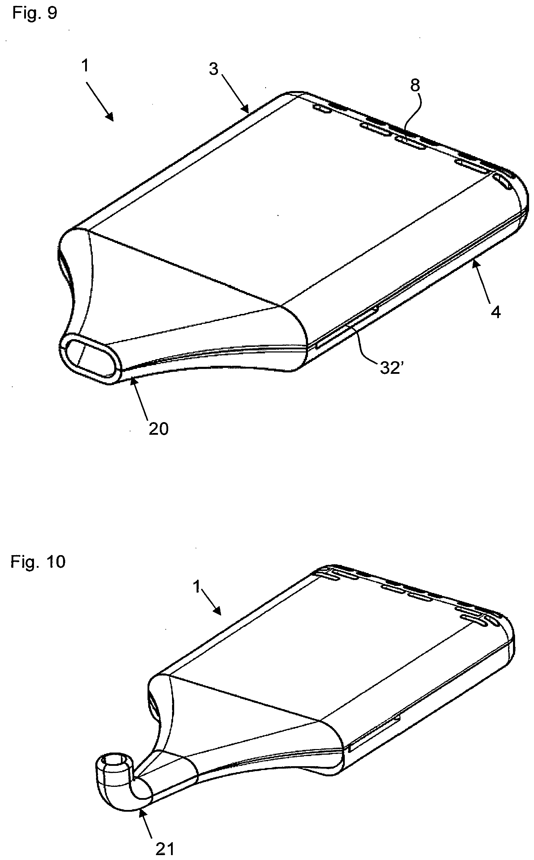

**Fig. 9** zeigt einen Pulverinhalator 1 in zusammengeklappter Anordnung, bei dem der Auslass wie in den Fig. 1 bis 8 als Mundstück 20 ausgeformt ist. Generell ist aber auch für beide Ausführungsformen des Pulverinhalators 1 denkbar, den Auslass als Nasenrohr 21 auszuformen, wie in **Fig. 10** dargestellt ist.

Alle erfindungsgemäßen Pulverinhalatoren 1 lassen sich grob in drei Teilbereiche gliedern:
- den Luftzufuhrbereich, der sich von der mit Lufteinlässen 8 versehenen Rückwand 31, also der Wand, die an der Basis der Halbschale 3 abgewandt von dem Auslassbereich vorliegt, bis zur Blisterkammer 11 erstreckt, und der den durch die Längsstege 6 begrenzten, trichterförmigen Zuluftkanal mit der Luftverwirbelungsstruktur 5 aufweist,
- den Pulverdepositions- und -freisetzungsbereich, der die Blisterkammer 11 mit der Pulveraufnahmemulde 9 umfasst, in die Wirkstoffmulde 101 eines Blisterelements 100 aufgenommen werden kann, und
- den Auslassbereich mit den jeweiligen Desagglomerationsstrukturen 17,17' und einem ausgeformtem Mund- oder Nasenstück.

Die erfindungsgemäße Gestaltung der drei Teilbereiche sorgt dafür, dass eine zur Applikation des Wirkstoffs, hierin alternativ auch als Medikament bezeichnet, beim Einatmen optimale Luftströmung gebildet wird, unabhängig davon, in welcher Weise der Patient einatmet. Auf diese Weise kann das Medikament optimal inhaliert werden und damit bestmögliche Wirkung zeigen.

Der Luftzufuhrbereich wird bei den in den Figuren gezeigten Beispielen durch den sich zu der Pulveraufnahmemulde 9 hin verjüngenden, von den Leitstegen 6 begrenzten Zuluftkanal gebildet, in den durch die Lufteinlässe 8 Luft einströmt, wenn der Patient mit dem in den Mund aufgenommenen Mundstück 20 einatmet. Die Lufteinlässe 8 sind in der Rückwand 31 der Halbschale 3 sowie an der Kontaktfläche der Rückwände 31,41 zwischen beiden Halbschalen 3,4 ausgebildet (vgl. **Fig. 5**). Der sich verjüngende Zuluftkanal und die jeweilige darin vorliegende Luftverwirbelungsstruktur 5 sorgen für Turbulenz und erhöhen die Geschwindigkeit des Zuluftstroms vor Eintritt in die Pulveraufnahmemulde 9. Natürlich kann die Anzahl der Zuluftkanäle und der darin angeordneten Luftverwirbelungsstrukturen in anderen Ausführungsformen, insbesondere auch in Ausführungsformen mit mehr als einer Pulveraufnahmemulde, durch entsprechende Erhöhung der Anzahl der Leitstege variiert werden.

Zur Anordnung des Blisters 100 (vgl. **Fig. 2**) weist die Halbschale 4 in einer Seitenwand 42 eine Aussparung 32' auf, durch die sich der eingelegte Blister 100 mit einem umgeschlagenen Überlapp 102 der Deckelfolie des Blisterelements 100 erstreckt. Luftdicht verschlossen wird diese Aussparung 32' durch den Überstand 32" an der anderen Halbschale 3. Die Wirkstoffmulde 101 des Blisterelements 100, die man auch als Kapsel bezeichnen könnte, findet in der in die Halbschale 4 eingeformten Mulde 9 Aufnahme. Zur positionsgenauen Anordnung des Blisters 100 sind ferner die Anschläge 12 in der Halbschale 4 vorgesehen.

Für einen unverrückbaren Halt des Blisterelements 100 in dem Pulverinhalator 1, wenn zum Öffnen der Wirkstoffmulde 101 an dem aus dem Pulverinhalator 1 ragenden Abschnitt 102 der Deckelfolie des Blisterelements 100 gezogen wird, wird das davon abgewandte Ende der Trägerplatte des Blisterelements 100 zwischen den Seitenwandabschnitten 32,42 der Halbschalen 3,4 gegenüber der Aussparung 32' und dem Überstand 32" aufgenommen. Hierzu ist dieser Abschnitt der Seitenwände 32,42 mit einer Riffelung 18 versehen.

Zur Führung der Luftströmung durch die eingelegte geöffnete Wirkstoffmulde 101 sind in der Halbschale 3 an entsprechender Stelle beidseitig der Pulveraufnahmemulde 9 bzw. der Wirkstoffmulde 101 angeordnete Führungswände 7 **(****Fig. 1****,** **Fig. 7****)** vorgesehen, die sich von den Längsstegen 6 erstrecken und den Luftstrom aus dem Lufteinlassbereich durch die Pulveraufnahmemulde 9 bzw. die darin angeordnete geöffnete, mit Pulver gefüllte Mulde 101 des Blisterelements 100 leiten.

An die Blisterkammer 11 schließt sich der Auslassbereich an, durch den das nun gebildete Aerosol aus Luft und Medikamentenpulver ausgelassen wird. Die Blisterkammer 11 wird im Auslassbereich durch die Desagglomerationsstrukturen 17, 17' abgegrenzt, durch die der nun mit Wirkstoff beladene Luftstrom nicht direkt zum Auslass gelangt, sondern durch die weitere Umlenkung Turbulenzen erzeugt werden, um Absetzten oder Agglomerieren des Pulvermedikaments zu verhindern. Zunächst allerdings sorgt die Querschnittsaufweitung nach der durch die Führungswände 7 eng begrenzten Passage durch die Pulveraufnahmemulde 9 für eine Reduktion der Strömungsgeschwindigkeit und erzielt damit einen Spacer-Effekt, d. h. die Wirkstoffpartikel werden gleichmäßig im Luftstrom verteilt. Die Desagglomeration der Wirkstoffpartikel hingegen wird anschließend durch in den Desagglomerationsstrukturen 17,17' in dem durch die Wandabschnitte 22 sich verjüngenden Auslassende mit Mundstück 20 unterstützt.

In den Figuren weisen die Pulverinhalatoren 1 jeweils drei Desagglomerationsstrukturen 17,17' auf, wobei in dem Ausführungsbeispiel in **Fig. 1 bis 4** eine Desagglomerationsstruktur 17 in der unteren Halbschale 4 und zwei Desagglomerationsstrukturen 17 in der oberen Halbschale 3 angeordnet sind. Generell ist es natürlich auch möglich mehr oder weniger Desagglomerationsstrukturen vorzusehen, diese können auch alle in einer der Halbschalen vorgesehen sein, oder anders auf beide Halbschalen verteilt sein.

Die bzw. jede Desagglomerationsstruktur 17,17' im Auslassbereich kann durch eine Gruppe aus ringförmig um einen Zylinder angeordneten Profilelementen, z. B. Lamellen, gebildet werden. Diese Lamellen können ein tragflächenähnliches Profil aufweisen, um die Luftströmung definiert zu verwirbeln.

In dem Ausführungsbeispiel nach **Fig. 5 bis 7** werden die drei Desagglomerationsstrukturen jeweils durch zwei Halbstrukturen 17' in jeder der Halbschalen 3,4 gebildet. Die Halbstrukturen 17' erstrecken sich lediglich bis zu der durch die Halbschalen 3,4 definierten Teilungsebene, so dass sich diese um das Drehgelenk 15 und Ausnehmung 15' verschwenken lassen. Generell ist in dieser Ausführungsform darauf zu achten, dass keine der Elemente und Strukturen im Inneren der Halbschalen 3,4 über die Teilungsebene ragt, so auch die Führungswände 7 und die Traversstege 53. Aus diesem Grund weist auch hier die Seitenwand 32 der Halbschale 3 gegenüber der Aussparung 32' an der Seitenwand 42 der Halbschale 4 keinen Überstand auf; die Aussparung 32' ist demzufolge mit einer geringeren Tiefe konzipiert, so dass die Halbschalen 3,4 bei eingelegtem Blisterelement an dieser Stelle dicht abschließen. Lediglich der Abschnitt der Seitenwand 32 oder Halbschale 3 mit der Riffelung 18 ragt über die Teilungsebene hinaus und der entsprechende Abschnitt mit der Riffelung 18 an der Seitenwand 42 der unteren Halbschale 4 ist entsprechend abgesenkt.

Mit dieser Anordnung ist ein Verschwenken der oberen Halbschale 3 zum Öffnen im Uhrzeigersinn und zum Schließen entgegen dem Uhrzeigersinn verbunden. Läge der geriffelte Überstand an der unteren Halbschale vor und ein entsprechend abgesenkter geriffelter Abschnitt an der oberen Halbschale, wären die Verschwenkungsrichtungen zum Öffnen und Schließen genau umgekehrt. Um dem Anwender die richtige Handhabung zu erleichtern, können entsprechende Informationen, beispielsweise Pfeilmarkierungen auf der Au-ßenseite der Halbschalen vorgesehen sein.

Ein erfindungsgemäßer Pulverinhalator 1 aus zwei Halbschalen 3 und 4 kann vorteilhaft rasch und günstig durch Spritzgießen eines Kunststoffes hergestellt werden. Alle erforderlichen Strukturen liegen in den Halbschalen vor bzw. sind im Falle der kegelstumpfförmigen Schnecke einstückig mit einer der Halbschalen verbunden. Der Zusammenbau der Halbschalen an dem Längsscharnier oder dem Drehgelenk ist unaufwändig, es sind keine weiteren Einzelteile erforderlich.

Vorteilhaft einfach lässt sich der Pulverinhalator 1 aus den zwei Halbschalen 3, 4 nach Einlegen eines Blisterelements 100 während des geöffneten Zustands und durch Zusammenklappen bzw. Verschwenken in betriebsbereiten Zustand überführen. Durch die flache Bauweise kann der Pulverinhalator 1 einfach und bequem mitgeführt werden, ohne dass Taschen an der Kleidung übermäßig ausgebeult werden. Wird die Einnahme des Medikaments erforderlich, ist nur noch das Abziehen des Endabschnitts 102 zur Öffnung der Pulvermulde 101 erforderlich, und das Medikament kann inhaliert werden.

Wie in **Fig. 1 bis 4** zu sehen ist, kann ferner ein zusätzlicher oder alternativer Öffnungsmechanismus für die Wirkstoffmulde 101 eines Blisterelements 100 vorgesehen sein, der einen als Dorn ausgebildeten Stempel 10 aufweist. Der Dorn 10 ist durch einen elastischen Einsatz 14 in der Halbschale 3 gehalten, der eine Wölbung 14' nach außen (siehe **Fig. 4**) aufweist, die dem Anwender den Druckpunkt zeigt, der betätigt werden muss, um eine eingelegte Blistermulde 101 mit dem Dorn 10 zu durchdringen. Nach Betätigung des Druckpunktes durch den Anwender wird der Dorn 10 durch den elastischen Einsatz 14 wieder in seine Anfangsposition zurückgestellt und lenkt zudem den Luftstrom in die Pulveraufnahmemulde bzw. die Wirkstoffmulde. Die Herstellung dieses Pulverinhalators kann erfolgen, indem zunächst der elastische Einsatz 14 gespritzt wird und die restliche Inhalatorform an den elastischen Einsatz 14 angespritzt wird oder umgekehrt, beispielsweise in einem 2K-Spritzgussverfahren. Selbstverständlich kann aber ein Pulverinhalator mit Längsscharnier und einlegbarer Luftverwirbelungsstruktur auch ohne Stempel ausgebildet werden, so dass das Öffnen der Wirkstoffmulde lediglich durch das Abziehen des aus dem Pulverinhalator ragenden Überlapps der Deckelfolie des Blisterelements erfolgt.

Ferner zeigt **Fig. 4** in der Längsschnittansicht, dass sich der Luftzufuhrkanal nicht nur in der Breite sondern auch in der Höhe in Richtung der Blisterkammer 11 verjüngt, wobei die Wandstärke der Halbschalen 3 und 4 gleichzeitig erhöht wird. Der durch die Lufteinlässe 8 eintretende Luftstrom wird entlang dem durch die Luftverwirbelungsstruktur 5 vorgegebenen schnecken- oder spiralförmigen Weg in Torsion versetzt (ähnlich einem Tornado). Durch die Drehung werden Fliehkräfte und Druckphänomene erzeugt, die wichtig für das vollständige Mitreißen des Pulvers aus der Pulveraufnahmemulde 9 sind.

## Patentansprüche

1. Pulverinhalator (1), umfassend zwei Halbschalen (3,4), die gelenkig miteinander verbindbar oder verbunden sind und in einer zusammengefügten Anordnung einen Lufteinlassbereich, einen Pulverdepositions- und -freigabebereich und einen Auslassbereich umschließen, durch die ein Fluidpfad verläuft, wobei
- in dem Lufteinlassbereich zumindest eine der Halbschalen (3,4) zumindest eine Lufteinlassöffnung (8) aufweist,
- in dem Pulverdepositions- und -freigabebereich eine der Halbschalen (3,4) zumindest eine Pulveraufnahmemulde (9) aufweist, in der eine Mulde (101) eines zur Aufnahme in dem Pulverinhalator (1) dimensionierten Blisterelements (100) aufnehmbar ist, das eine Trägerfolie aufweist, die zumindest die Mulde (101) aufweist, die zur Aufnahme zumindest eines inhalierbaren Wirkstoffpulvers ausgebildet ist, wobei eine Deckelplatte die Mulde (101) verschließt, die in Größe und Position entsprechend zur Aufnahme in der zumindest einen Pulveraufnahmemulde (9) des Pulverinhalators (1) ausgebildet ist, und
- der Auslassbereich zumindest eine Desagglomerationsstruktur (17,17') und einen Auslass für Aerosol aufweist, der durch die Halbschalen (3,4) ausgebildet wird, wobei die zwei Halbschalen (3,4) flach sind und eine längliche im Wesentlichen rechteckige, trapezförmige, tropfenförmige oder ovale Basis aufweisen, die durch eine Wand oder Wandabschnitte berandet ist, und wobei
- die zumindest eine Lufteinlassöffnung (8) an einem von der Auslassseite abgewandten Wandabschnitt (31,41) angeordnet ist,
- an zumindest einer der zwei Halbschalen (3,4) zumindest zwei Leitstege (6) vorliegen, die sich von dem von der Auslassseite abgewandten Wandabschnitt (31.41) bis hin zu der zumindest einen Pulveraufnahmemulde (9) erstrecken,
- sich die Leitstege (6) der zumindest einen Halbschale (3,4) in ihrer Höhe bis zu der anderen Halbschale (3,4) oder bis zu den Leitstegen (6) der anderen Halbschale (3,4) erstrecken, wobei jeweils zwei nebeneinander benachbarte Leitstege (6) einen Zuluftkanal ausbilden, der zu der zumindest einen Pulveraufnahmemulde (9) einen sich verjüngenden Querschnitt aufweist,
- die zumindest eine Pulveraufnahmemulde (9) entlang dem Fluidpfad beidseitig durch Führungswände (7) eingefasst ist, die sich von den äußersten zu der zumindest einen Pulvermulde (9) führenden Leitstegen (6) erstrecken,
wobei in dem zumindest einen Zuluftkanal zumindest eine Luflverwirbelungsslruktur (5) vorliegt, die den Fluidpfad zwischen der zumindest einen Lufleinlassöffnung (8) und dem Pulverdepositions- und -freigabebereich definiert, und wobei der Pulverinhalator mindestens eins der folgenden Merkmale A und B aufweist:
A. in dem Pulverdepositions- und -freigabebereich weist die Halbschale (3,4), die nicht die zumindest einen Pulveraufnahmemulde (9) aufweist, an einer der zumindest einen Pulveraufnahmemulde (9) gegenüberliegenden Stelle einen Stempel (10) auf, der mittels eines elastischen Einsatzes (14) in dieser Halbschale (3,4) in Richtung der zumindest einen Pulveraufnahmemulde (9) bewegbar eingesetzt ist, wobei der Stempel (10) als Dorn zur Öffnung einer Wirkstoffmulde (101) eines in den Pulverinhalator (1) eingelegten Biistereiements (100) ausgebildet ist; und
B. an einer der Halbschalen (3,4) weist einer der Seitenwandabschnitte (32,42) auf Höhe der Pulveraufnahmemulde (9) eine Aussparung (32') auf, deren Breite und Höhe einer Breite und Höhe eines zum Einlegen in den Pulverinhalator (1) vorbestimmten Blisterelements (100) entsprechen, wobei die zweite Halbschale (3,4), die nicht die Aussparung (32') hat, an dem Seitenwandabschnitt, der bei einer zusammengefügten Anordnung der Halbschalen (3,4) über der Aussparung (32') zu liegen kommt, einen Überstand (32") aufweist, der dazu ausgebildet ist, die Aussparung (32') zu verschließen, wenn ein Blisterelement (100) in dem Pulverinhalator (1) aufgenommen ist.

2. Pulverinhalator (1) nach Anspruch 1, **dadurch gekennzeichnet, dass** der Pulverinhalator (1) zumindest zwei Pulveraufnahmemulden (9) aufweist, wobei jeweils zumindest ein Zuluftkanal zu jeder der Pulveraufnahmemulden (9) führt.

3. Pulverinhalator (1) nach zumindest einem der Ansprüche 1 bis 2, **dadurch gekennzeichnet, dass** die Luftverwirbelungsstruktur (5) durch
- einen elliptischen Kegelstumpf (51) mit zumindest einer wendelartig entlang seiner Mantelfläche verlaufenden Erhebung (52) gebildet wird, der in dem Luftzufuhrkanal zwischen zwei benachbarten Leitstegen (6) angeordnet wird, oder
- durch mehrere Traversstege (53) gebildet wird, die zwischen benachbarten Leitstegen (6) verlaufen, wobei die Traversstege (53) in beiden Halbschalen (3,4) derart angeordnet sind, dass sie in der zusammengefügten Anordnung gegensinnig verlaufen.

4. Pulverinhalator (1) nach Anspruch 3,
**dadurch gekennzeichnet, dass**
der Kegelstumpf (51) über seine Basisfläche an dem von der Auslassseite abgewandten Wandabschnitt (31,41) einer der Halbschalen (3,4) angelenkt ist.

5. Pulverinhalator (1) nach Anspruch 3 oder 4,
**dadurch gekennzeichnet, dass**
der Kegelstumpf (51) durch zumindest ein Filmscharnier (16) an dem von der Auslassseite abgewandten Wandabschnitt (31,41) einer der Halbschalen (3,4) angelenkt ist.

6. Pulverinhalator (1) nach zumindest einem der Ansprüche 1 bis 5,
**dadurch gekennzeichnet, dass**
die gelenkige Verbindung der beiden Halbschalen (3,4) durch ein Scharnier (15") an benachbarten Seitenwänden (32,42) oder durch ein Schwenkgelenk (15,15') bereitgestellt wird.

7. Pulverinhalator (1) nach Anspruch 6,
**dadurch gekennzeichnet, dass**
das Schwenkgelenk (15,15') im Bereich zwischen einer Seitenwand (32,42) und dem von der Auslassseite abgewandten Wandabschnitt (31,41) angeordnet ist.

8. Pulverinhalator (1) nach zumindest einem der Ansprüche 1 bis 7,
**dadurch gekennzeichnet, dass**
die zumindest eine Desagglomerationsstruktur (17) im Auslassbereich durch eine Gruppe aus ringförmig um eine Zylinderstruktur angeordneten Profilelementen gebildet wird, die sich zwischen den beiden Halbschalen (3,4) erstreckt, wobei
- die zumindest eine Desagglomerationsstruktur (17) an einer der beiden Halbschalen (3,4) angeordnel ist oder wobei mehrere Desagglomerationsstrukturen (17) an unterschiedlichen Stellen im Auslassbereich auf beide Halbschalen (3,4) verteilt sind,
oder wobei
- die zumindest eine Desagglomerationsstruktur (17) durch jeweils zwei Halbstrukturen (17') gebildet wird, von denen jeweils eine Halbstruktur (17') an jeder Halbschale (3,4) angeordnet ist, so dass die Halbstrukturen (17') in der zusammengefügten Anordnung der Halbschalen (3,4) die sich zwischen beiden Halbschalen (3,4) erstreckende Desagglomerationsstruktur (17) bilden.

9. Pulverinhalator (1) nach zumindest einem der vorstehenden Ansprüche,
**dadurch gekennzeichnet, dass**
die Halbschale (3,4), die die Pulveraufnahmemulde (9) aufweist, zumindest einen Anschlag (12) aufweist, der zur Berandung des zur Aufnahme in dem Pulverinhalator (1) vorbestimmten Blisterelements (100) angeordnet ist.

10. Pulverinhalator (1) nach zumindest einem der vorstehenden Ansprüche,
**dadurch gekennzeichnet, dass**
beide Halbschalen (3,4) an den Seitenwandabschnitten (32,42) auf Höhe der Pulveraufnahmemulde (9) gegenüber der Aussparung (32') und gegenüber dem Überstand (32") eine Riffelung (18) aufweisen, deren Breite einer Breite eines zum Einlegen in den Pulverinhalator (1) vorbestimmten Blisterelements (100) entspricht,
oder dass
- die Halbschale (3,4), die die Pulveraufnahmemulde (9) aufweist, einen Überstand aufweist, der dazu ausgebildet ist, mit einer korrespondierenden Ausnehmung des zur Aufnahme in dem Pulverinhalator (1) vorbestimmten Blisterelements (100) in Eingriff zu kommen.

11. Pulverinhalator (1) nach Anspruch 10, **dadurch gekennzeichnet, dass**
der Überstand, der dazu ausgebildet ist, mit einer korrespondierenden Ausnehmung des zur Aufnahme in dem Pulverinhalator (1) vorbestimmten Blisterelements (100) in Eingriff zu kommen, ein kuppelförmiger Überstand ist.

12. Pulverinhalator (1) nach zumindest einem der vorstehenden Ansprüche,
**dadurch gekennzeichnet, dass** an den Halbschalen (3,4) Rastmittel zum lösbaren Zusammenfügen der beiden Halbschalen (3,4) vorgesehen sind.

13. Pulverinhalationsset (1,100)
**dadurch gekennzeichnet, dass**
es einen Pulverinhalator (1) nach zumindest einem der Ansprüche 1 bis 12 und ein zur Aufnahme in dem Pulverinhalator (1) dimensioniertes Blisterelement (100) aufweist, wobei das Blisterelement (100) eine Trägerfolie mit einer zumindest einen Mulde (101) aufweist, die zur Aufnahme zumindest eines inhalierbaren Wirkstoffpulvers ausgebildet ist, und eine Deckelplatte, die die Mulde (101) verschließt, wobei die zumindest eine Mulde (101) des Blisterelements (100) in Größe und Position entsprechend zur Aufnahme in der zumindest einen Pulveraufnahmemulde (9) des Pulverinhalators (1) ausgebildet ist.

14. Pulverinhalationsset (1,100) nach Anspruch 13,
**dadurch gekennzeichnet, dass**
die Deckelplatte zum Öffnen der Mulde (101) von der Trägerplatte durch eine Aussparung (32') in einem Seitenwandabschnitt (32,42) auf Höhe der Pulveraufnahmemulde (9) des Pulverinhalators (1) ablösbar ausgebildet ist,
und/oder
die Halbschale (3,4) des Pulverinhalators (1). die nicht die zumindest eine Pulveraufnahmemulde (9) aufweist, einen gegenüber der Pulveraufnahmemulde (9) angeordneten, elastisch bewegbaren und als Dorn ausgebildeten Stempel (10) zum Durchdringen der Deckelplatte des Blisterelements (100) aufweist.

## Claims

1. Powder inhaler (1), comprising
- two half shells (3, 4) connectable or connected to each other in an articulated manner and, in a joined arrangement, enclosing an air inlet area, a powder deposition and release area, and an outlet area through which a fluid path is extending, wherein
- in the air inlet area at least one of the half shells (3, 4) comprises at least one air inlet opening (8),
- in the powder deposition and release area, one of the half shells (3, 4) comprises at least one powder receiving depression (9), in which a depression (101) of a blister element (100) dimensioned for reception in the powder inhaler (1) can be received, which comprises a support film comprising at least the depression (101) that is formed for receiving at least one active ingredient powder that is inhalable, wherein a cover plate of the blister element (100) closes off the depression (101), that in size and position is formed correspondingly for reception in the at least one powder receiving depression (9) of the powder inhaler (1)
- the outlet area comprises at least one de-agglomeration structure (17, 17') and an outlet for aerosol that is formed by the half shells (3, 4), wherein the two half shells (3, 4) are flat and comprise an elongate, substantially rectangular, trapezoidal, drop-shaped or oval base that is rimmed by a wall or wall sections, and wherein
- the at least one air inlet opening (8) is arranged on a wall section (31, 41) facing away from the outlet area
- on at least one of the two half shells (3, 4) at least two guide webs (6) are present and are extending from the wall section (31, 41) facing away from the outlet area all the way to the at least one powder receiving depression (9)
- the guide webs (6) of the at least one half shell (3, 4) in regard to their height extend all the way to the other half shell (3, 4) or to the guide webs (6) of the other half shell (3, 4) for forming at least one air supply channel, wherein two side by side neighboring guide webs (6) form an air supply channel, respectively, whose cross-section is tapering in the direction toward the powder receiving depression (9)
- guide walls (7) frame the at least one powder receiving depression (9) along the fluid path on both sides and extend away from the outermost guide webs (6) extending to the at least one powder depression (9),
wherein at least one air swirling structure (5) is present in the at least one air supply channel and defines the fluid path between the at least one air inlet opening (8) and the powder deposition and release area and wherein the powder inhaler (1) comprises at least one of the following features A and B:
A. the half shell (3, 4) that does not comprise the at least one powder receiving depression (9) comprises a plunger (10) in the powder deposition and release area at a location which is positioned opposite the at least one powder receiving depression (9), wherein the plunger (10) is inserted in this half shell (3, 4) so as to be moveable in the direction toward the at least one powder receiving depression (9) by means of an elastic insert (14) and is formed as a pricker for opening an active ingredient depression (101) of a blister element (100) inserted into the powder inhaler (1); and
B. of one of the sidewall sections (32, 42) on one of the half shells (3, 4) comprises a cutout (32') at the level of the powder receiving depression (9), the width and height of the cutout (32') corresponding to a width and height of a blister element (100) predetermined for insertion into the powder inhaler (1), wherein the second half shell (3, 4) that does not have the cutout (32') comprises a projection (32") on the sidewall section (32, 42) that, in a joined arrangement of the half shells (3, 4), is positioned above the cutout (32'), wherein the projection (32") is designed to close off the cutout (32') when a blister element (100) is received in the powder inhaler (1).

2. Powder inhaler (1) according to claim 1,
**characterized in that**
the powder inhaler (1) comprises at least two powder receiving depressions (9), wherein at least one air inlet channel extends to each one of the powder receiving depressions (9), respectively.

3. Powder inhaler (1) according to at least one of the claims 1 to 2,
**characterized in that**
the air swirling structure (5) is formed by
- an elliptical truncated cone (51) to be arranged in the air supply channel between two neighboring guide webs (6) and comprising at least one projection (52) that extends in a spiral shape along its wall surface, or
- by several transverse stays (53) that extend between neighboring guide webs (6), wherein the transverse stays (53) are arranged in both half shells (3, 4) in such a way that in the joined arrangement they extend oppositely oriented relative to each other.

4. Powder inhaler (1) according to claim 3,
**characterized in that**
the truncated cone (51) is connected in an articulated manner by its base surface to the wall section (31, 41) of one of the half shells (3, 4) that is facing away from the outlet side.

5. Powder inhaler (1) according to claim 3 or 4,
**characterized in that**
the truncated cone (51) is connected by at least one film hinge (16) to the wall section (31, 41) of one of the half shells (3, 4) that is facing away from the outlet side.

6. Powder inhaler (1) according to at least one of the claims 1 to 5,
**characterized in that**
the articulated connection of the two half shells (3, 4) is provided by a hinge (15") on neighboring sidewalls (32, 42) or by a pivot joint (15, 15').

7. Powder inhaler (1) according to claim 6,
**characterized in that**
the pivot joint (15, 15') is arranged in the area between a sidewall (32, 42) and the wall section (31, 41) facing away from the outlet side.

8. Powder inhaler (1) according to at least one of the claims 1 to 7,
**characterized in that**
the at least one de-agglomeration structure (17) in the outlet area is formed by a group of profiled elements arranged in a ring shape about a full cylinder structure that extends between the two half shells (3, 4), wherein
- the at least one de-agglomeration structure (17) is arranged on one of the two half shells (3, 4) or wherein several de-agglomeration structures (17) are distributed at different locations in the outlet area on both half shells (3, 4), or wherein
- the at least one de-agglomeration structure (17) is formed by two half structures (17'), respectively, of which one half structure (17') is arranged on each half shell (3, 4), respectively, so that the half structures (17') in the joined arrangement of the half shells (3, 4) form the de-agglomeration structure (17) extending between both half shells (3, 4).

9. Powder inhaler (1) according to at least one of the preceding claims,
**characterized in that**
the half shell (3, 4) that comprises the powder receiving depression (9) comprises at least one stop (12) that is arranged for framing the blister element (100) predetermined for reception in the powder inhaler (1).

10. Powder inhaler (1) according to at least one of the preceding claims,
**characterized in that**
both half shells (3, 4) comprise a ribbing (18) at the sidewall sections (32, 42) at the level of the powder receiving depression (9) opposite the cutout (32') and opposite the projection (32"), the width of the ribbing corresponding to a width of a blister element (100) predetermined for reception in the powder inhaler (1),
or **in that**
the half shell (3, 4) which comprises the powder receiving depression (9) comprises a projection that is designed to engage a corresponding cutout of the blister element (100) predetermined for reception in the powder inhaler (1).

11. Powder inhaler (1) according to claim 10,
**characterized in that**
the projection that is designed to engage a corresponding cutout of the blister element (100) predetermined for reception in the powder inhaler (1) is a dome-shaped projection.

12. Powder inhaler (1) according to at least one of the preceding claims,
**characterized in that**
on the half shells (3, 4) locking means for releasably joining the two half shells (3, 4) are provided.

13. Powder inhalation set (1, 100)
**characterized in that**
it comprises a powder inhaler (1) according to at least one of the claims 1 to 12 and a blister element (100) dimensioned for reception in the powder inhaler (1), wherein the blister element (100) comprises a support film with at least one depression (101) that is formed for receiving at least one active ingredient powder that is inhalable, and a cover plate that closes off the depression (101), wherein the at least one depression (101) of the blister element (100) in size and position is formed correspondingly for reception in the at least one powder receiving depression (9) of the powder inhaler (1).

14. Powder inhalation set (1, 100) according to claim 13,
**characterized in that,**
for opening the depression (101), the cover plate is designed to be removable from the support plate through a cutout (32') in a sidewall section (32, 42) at the level of the powder receiving depression (9) of the powder inhaler (1), and/or
the half shell (3, 4) that does not comprise the at least one powder depression (9) comprises a plunger (10), which is arranged opposite the powder receiving depression (9), is elastically moveable, and designed as a pricker to penetrate the cover plate of the blister element (100).

## Revendications

1. Inhalateur de poudre (1) comprenant deux demi-coques (3, 4) qui peuvent être connectées ou sont connectées l'une à l'autre de manière articulée et enferment dans un agencement assemblé une région d'entrée d'air, une région de dépôt et de libération de poudre et une région de sortie à travers lesquelles un parcours de fluide s'étend, dans lequel
- au moins une des demi-coques (3, 4) présente au moins une ouverture d'entrée d'air (8) dans la région d'entrée d'air,
- une des demi-coques (3, 4) présente au moins un moule de réception de poudre (9) dans la région de dépôt et de libération de poudre dans lequel un moule (101) d'un élément d'emballage-coque (100) dimensionné pour la réception dans l'inhalateur de poudre (1) peut être reçu, lequel élément présente une feuille support qui présente au moins le moule (101) qui est réalisé pour la réception d'au moins une poudre de substance active pouvant être inhalée, dans lequel une plaque de couvercle ferme le moule (101), laquelle est réalisée en termes de taille et de position pour correspondre à la réception dans l'au moins un moule de réception de poudre (9) de l'inhalateur de poudre (1), et
- la région de sortie présente au moins une structure de désagglomération (17, 17') et une sortie pour de l'aérosol qui est réalisée par les demi-coques (3, 4), dans lequel les deux demi-coques (3, 4) sont plates et présentent une base longitudinale essentiellement rectangulaire, trapézoïdale, en forme de goutte ou ovale qui est bordée par une paroi ou des sections de paroi, et dans lequel
- l'au moins une ouverture d'entrée d'air (8) est disposée sur une section de paroi (31, 41) détournée du côté de sortie,
- au moins deux entretoises directrices (6) sont présentes sur au moins une des deux demi-coques (3, 4), lesquelles s'étendent de la section de paroi (31, 41) détournée du côté de sortie jusqu'à l'au moins un moule de réception de poudre (9),
- les entretoises directrices (6) de l'au moins une demi-coque (3, 4) s'étendent dans leur hauteur jusqu'à l'autre demi-coque (3, 4) ou jusqu'aux entretoises directrices (6) de l'autre demi-coque (3, 4), dans lequel deux entretoises directrices (6) voisines côte à côte réalisent chacune un canal d'air frais qui présente une section transversale qui se rétrécit vers l'au moins un moule de réception de poudre (9),
- l'au moins un moule de réception de poudre (9) est délinéé le long du parcours de fluide des deux côtés par des parois de guidage (7) qui s'étendent des entretoises directrices (6) les plus à l'extérieur menant à l'au moins un moule de poudre (9), dans lequel au moins une structure de tourbillonnement d'air (5) est présente dans l'au moins un canal d'air frais, laquelle définit le parcours de fluide entre l'au moins une ouverture d'entrée d'air (8) et la région de dépôt et de libération de poudre, et dans lequel l'inhalateur de poudre présente au moins une des caractéristiques A et B suivantes:
A. la demi-coque (3, 4) qui ne présente pas l'au moins un moule de réception de poudre (9) présente dans la région de dépôt et de libération de poudre sur un côté opposé à l'au moins un moule de réception de poudre (9) un poinçon (10) qui est inséré dans cette demi-coque (3, 4) de manière mobile en direction de l'au moins un moule de réception de poudre (9) au moyen d'un insert élastique (14), dans lequel le poinçon (10) est réalisé en tant que mandrin pour l'ouverture d'un moule de substance active (101) d'un élément d'emballage-coque (100) intercalé dans l'inhalateur de poudre (1); et
B. une des sections de paroi latérales (32, 42) présente sur une des demi-coques (3, 4) à hauteur du moule de réception de poudre (9) un évidement (32') dont la largeur et hauteur correspondent à une largeur et hauteur d'un élément d'emballage-coque (100) prédéterminé pour l'intercalage dans l'inhalateur de poudre (1), dans lequel la seconde demi-coque (3, 4) qui n'a pas l'évidement (32') présente sur la section de paroi latérale qui vient reposer au-dessus de l'évidement (32') dans le cas d'un agencement assemblé des demi-coques (3, 4) une saillie (32") qui est réalisée pour fermer l'évidement (32') lorsqu'un élément d'emballage-coque (100) est reçu dans l'inhalateur de poudre (1).

2. Inhalateur de poudre (1) selon la revendication 1,
**caractérisé en ce que**
l'inhalateur de poudre (1) présente au moins deux moules de réception de poudre (9), dans lequel au moins un canal d'air frais mène à chaque fois à chacun des moules de réception de poudre (9).

3. Inhalateur de poudre (1) selon au moins une des revendications 1 à 2,
**caractérisé en ce que**
la structure de tourbillonnement d'air (5) est formée par
- un cône tronqué elliptique (51) avec au moins un rehaussement (52) s'étendant de manière hélicoïdale le long de sa face d'enveloppe qui est disposé dans le canal d'amenée d'air entre deux entretoises directrices voisines (6), ou
- est formée par plusieurs entretoises transversales (53) qui s'étendent entre des entretoises directrices voisines (6), dans lequel les entretoises transversales (53) sont disposées dans les deux demi-coques (3, 4) de telle sorte qu'elles s'étendent en sens inverse dans l'agencement assemblé.

4. Inhalateur de poudre (1) selon la revendication 3,
**caractérisé en ce que**
le cône tronqué (51) est articulé par le biais de sa face de base à la section de paroi (31, 41) détournée du côté de sortie d'une des demi-coques (3, 4).

5. Inhalateur de poudre (1) selon la revendication 3 ou 4,
**caractérisé en ce que**
le cône tronqué (51) est articulé par au moins une charnière intégrale (16) à la section de paroi (31, 41) détournée du côté de sortie d'une des demi-coques (3, 4).

6. Inhalateur de poudre (1) selon au moins une des revendications 1 à 5,
**caractérisé en ce que**
la connexion articulée des deux demi-coques (3, 4) est mise à disposition par une charnière (15") au niveau de parois latérales voisines (32, 42) ou par une articulation pivotante (15, 15').

7. Inhalateur de poudre (1) selon la revendication 6,
**caractérisé en ce que**
l'articulation pivotante (15, 15') est disposée dans la région entre une paroi latérale (32, 42) et la section de paroi (31, 41) détournée du côté de sortie.

8. Inhalateur de poudre (1) selon au moins une des revendications 1 à 7,
**caractérisé en ce que**
l'au moins une structure de désagglomération (17) est formée dans la région de sortie par un groupe d'éléments profilés disposés de manière annulaire autour d'une structure cylindrique qui s'étend entre les deux demi-coques (3, 4), dans lequel
- l'au moins une structure de désagglomération (17) est disposée sur une des deux demi-coques (3, 4) ou dans lequel plusieurs structures de désagglomération (17) sont réparties à différents endroits dans la région de sortie sur les deux demi-coques (3, 4), ou dans lequel
- l'au moins une structure de désagglomération (17) est formée par deux demi-structures (17') à chaque fois parmi lesquelles une demi-structure (17') est à chaque fois disposée sur chaque demi-coque (3, 4) de sorte que les demi-structures (17') forment dans l'agencement assemblé des demi-coques (3, 4) la structure de désagglomération (17) s'étendant entre les deux demi-coques (3, 4).

9. Inhalateur de poudre (1) selon au moins une des revendications précédentes,
**caractérisé en ce que**
la demi-coque (3, 4) qui présente le moule de réception de poudre (9) présente au moins une butée (12) qui est disposée pour délinéer l'élément d'emballage-coque (100) prédéterminé pour la réception dans l'inhalateur de poudre (1).

10. Inhalateur de poudre (1) selon au moins une des revendications précédentes,
**caractérisé en ce que**
les deux demi-coques (3, 4) présentent sur les sections de paroi latérales (32, 42) à hauteur du moule de réception de poudre (9) à l'opposé de l'évidement (32') et à l'opposé de la saillie (32") un striage (18) dont la largeur correspond à une largeur d'un élément d'emballage-coque (100) prédéterminé pour l'intercalage dans l'inhalateur de poudre (1),
ou que
- la demi-coque (3, 4) qui présente le moule de réception de poudre (9) présente une saillie qui est réalisée pour s'engrener avec un creux correspondant de l'élément d'emballage-coque (100) prédéterminé pour la réception dans l'inhalateur de poudre (1).

11. Inhalateur de poudre (1) selon la revendication 10,
**caractérisé en ce que**
la saillie qui est réalisée pour s'engager avec un creux correspondant de l'élément d'emballage-coque (100) prédéterminé pour la réception dans l'inhalateur de poudre (1) est une saillie en forme de coupole.

12. Inhalateur de poudre (1) selon au moins une des revendications précédentes,
**caractérisé en ce que**
des moyens d'encliquetage pour l'assemblage amovible des deux demi-coques (3, 4) sont prévus sur les demi-coques (3, 4).

13. Kit d'inhalation de poudre (1, 100),
**caractérisé en ce que**
il présente un inhalateur de poudre (1) selon au moins une des revendications 1 à 12 et un élément d'emballage-coque (100) dimensionné pour la réception dans l'inhalateur de poudre (1), dans lequel l'élément d'emballage-coque (100) présente une feuille support avec au moins un moule (101) qui est réalisé pour la réception d'au moins une poudre de substance active pouvant être inhalée, et une plaque de couvercle qui ferme le moule (101), dans lequel l'au moins un moule (101) de l'élément d'emballage-coque (100) est réalisé en termes de taille et de position pour correspondre à la réception dans l'au moins un moule de réception de poudre (9) de l'inhalateur de poudre (1).

14. Kit d'inhalation de poudre (1, 100) selon la revendication 13,
**caractérisé en ce que**
la plaque de couvercle pour l'ouverture du moule (101) de la plaque de support est réalisée de manière amovible par un évidement (32') dans une section de paroi latérale (32, 42) à hauteur du moule de réception de poudre (9) de l'inhalateur de poudre (1),
et/ou
la demi-coque (3, 4) de l'inhalateur de poudre (1) qui ne présente pas l'au moins un moule de réception de poudre (9) présente un poinçon (10) disposé à l'opposé du moule de réception de poudre (9), mobile de manière élastique et réalisé en tant que mandrin pour transpercer la plaque de couvercle de l'élément d'emballage-coque (100).
